# EUROPEAN PATENT APPLICATION

(11) **EP 4 349 845 A1**
(43) Date of publication of application: **10.04.2024**
(21) Application number: 22815625.3
(22) Date of filing: 14.03.2022
(51) Int. Cl.: C07H 17/07, A61P 17/00, A61Q 19/00, A61K 36/48, A61K 8/60, A61K 31/7048, A23L 2/52, A23L 33/105

(54) **NOVEL ISOFLAVONE COMPOUND**

(30) Priority: 03.06.2021 JP 2021093328
(71) Applicant: Nabocul Cosmetics Co., Ltd., Tokyo 101-0051 (JP)
(72) Inventor: TSIM, Karl Wah-Keung, Hong Kong (CN); YASHIRO, Takuya, Tokyo 101-0051 (JP); DONG, Tina Ting-Xia, Hong Kong (CN); PENG, Zhi-Tian, Shenzhen (CN); WANG, Huai-You, Shenzhen (CN); LI, Yong, Tokyo 101-0051 (JP)
(74) Representative: Vossius & Partner Patentanwälte Rechtsanwälte mbB
(86) International application number: PCT/JP2022/011173
(87) International publication number: WO 2022/254868

(57) **Abstract**

Provided is a novel active ingredient derived from locust bean and the use thereof. The present invention is a compound represented by the following formula (I) or a salt thereof, or a solvate thereof.

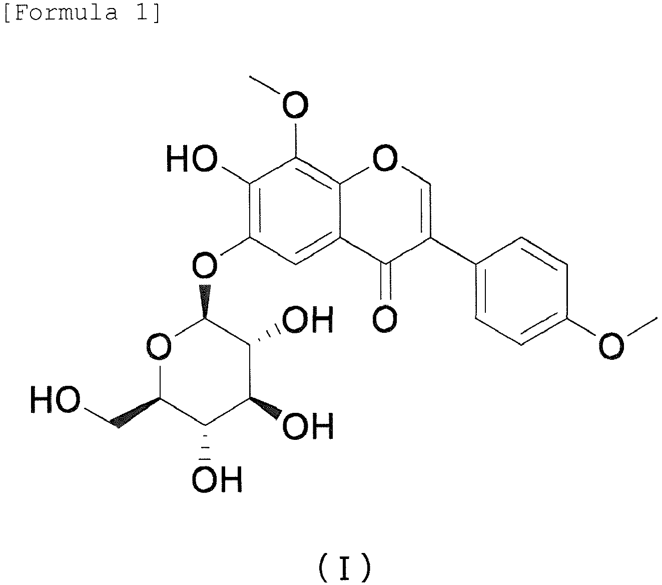

## Description

### Technical Field

The present invention relates to a novel isoflavone compound obtained by separation and purification from locust bean (*Ceratonia siliqua* L.) and the use of the compound as well as a method for producing the compound.

### Background Art

Locust bean (*Ceratonia siliqua L*.) is a leguminous plant mainly native to the Mediterranean region. The pod, that is, a shell and fruits, of locust bean is referred to as a carob and has been long used as food or a food ingredient. A mature pod has a length of about 10 to 25 cm and a sweet taste. The pod of locust bean contains a large amount of polysaccharides, cellulose and minerals, and a small amount of proteins and non-carbohydrate-based low-molecular compounds, and the like. In recent years, research on new functions of locust bean has been in progress, and it has been reported that an aqueous extract of pods of locust bean has multiple pharmacological actions such as antidiarrheal, antioxidant, antimicrobial, antiulcer and anti-inflammatory actions in the gastrointestinal tract, and that it has preventive and therapeutic effects on gastrointestinal diseases such as ulcerative colitis and gastric ulcer (Non Patent Literatures 1 and 2). In addition, Patent Literature 1 discloses that a seed extract of locust bean has an α-glucosidase inhibitory activity and thereby has the effect of suppressing the body weight gain, and Patent literature 2 discloses that peptides extracted from seeds of locust bean activate the expression of aquaporins.

Inflammation is a very common and important pathological process and is closely related to most diseases such as neurodegenerative diseases, cancers, cardiovascular disease, rheumatoid arthritis, respiratory diseases, diabetes and muscle and soft tissue injuries. Inflammation has been identified as one of the important pathogenicity factors in many acute and chronic diseases. IL-6 (interleukin-6) is a glycoprotein having a molecular weight of 21 to 28 kDa, and is one of the inflammatory cytokines involved in biological processes, such as acute inflammation, tissue damage and immune response. IL-6 is produced mainly when TNF-α and IL-1 induce monocytes. IL-6 not only activates inflammatory cells, but also induces the expression of acute-phase proteins, catalyzes and amplifies inflammatory responses and toxic actions, and damages tissue cells. For this reason, an IL-6 production inhibition assay has been used as a means of screening anti-inflammatory agents. Many clinical therapeutic drugs for inflammatory diseases have various adverse effects and drug resistances, which limits their clinical application. Therefore, novel anti-inflammatory agents having a high therapeutic effect, few adverse effects and high safety have been still desired.

In the meantime, aquaporin 3 (AQP3) is one of the aquaporin isoforms most highly expressed in the human skin. Aquaporin 3, which is present on the cell membrane, is a transporter transporting small molecules such as water through the membrane. It mediates membrane transport of small molecules such as water, glycerol and urea in a living organism by forming "pores" in the cell membrane, and has the effect of adjusting water balance. Aquaporin 3 is involved in skin moisture, skin barrier function and wound healing, and has the function of keeping morphology and function of the skin normal. It is related to proliferation and migration, and even early differentiation, of keratinocytes, as well as many skin diseases such as atopic dermatitis, vitiligo, skin tumors and psoriasis. A decrease in the expression level of aquaporin 3 (downregulation) is known to cause natural aging of the non-exposed skin.

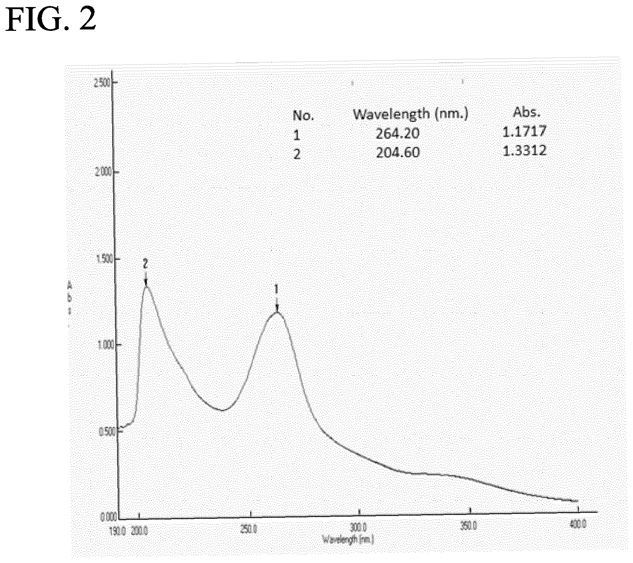

Filaggrin 2 (FLG2) is a member of the S100 fused-type protein family, which is present in the epidermal differentiation complex (EDC). Filaggrin 2 protein and the mRNA of filaggrin 2 are localized mainly in the keratinized epithelium, especially in the skin. The expression pattern of filaggrin 2 is restricted to the upper stratum granulosum and the stratum corneum, is similar to that of filaggrin, and is structurally closely related to filaggrin. Filaggrin 2 is involved in epithelial homeostasis. In addition, it is also required for skin keratinization and also has the function of protecting the skin barrier. Defects in the filaggrin 2 gene are related to the onset of skin diseases.

### Citation List

### Patent Literature

Patent Literature 1
   Japanese Patent Laid-Open No. 2005-119999
Patent Literature 2
   Japanese Patent Laid-Open No. 2013-515704

### Non Patent Literature

Non Patent Literature 1
   Rtibi K, Jabri M A, Selmi S, et al., "Preventive effect of carob (Ceratonia siliqua L.) in dextran sulfate sodium-induced ulcerative colitis in rat", RSC Advances, 2016, Vol. 6, p.19992-20000.
Non Patent Literature 2
   Rtibi K, Selmi S, Grami D, et al., "Chemical constituents and pharmacological actions of carob pods and leaves (Ceratonia siliqua L.) on the gastrointestinal tract: A review", Biomedicine & Pharmacotherapy, 2017, Vol. 93, p.522-528.

### Summary of Invention

### Technical Problem

However, Non Patent Literatures 1 and 2 report that the extract of pods of locust bean has pharmacological actions such as antidiarrheal, antioxidant, antimicrobial, antiulcer and anti-inflammatory actions in the gastrointestinal tract and has therapeutic effects on gastrointestinal diseases, and Patent Literatures 1 and 2 report that the seed extract of locust bean has the effect of suppressing the body weight gain, and that peptides activating the expression of aquaporins are included, but a specific active ingredient thereof has not yet been identified.

In addition, the use of pods instead of seeds (beans) of locust bean for improving the skin barrier function through enhanced expression of aquaporin 3 gene or filaggrin 2 gene has not been previously studied, and its effectiveness has been completely unclear.

Accordingly, the present invention has been made in view of the above points. An object of the present invention is to provide a novel active ingredient derived from locust bean and the use thereof.

Another object of the present invention is to provide a novel anti-inflammatory agent, derived from locust bean, which is capable of inhibiting IL-6 production. A further object of the present invention is to provide a skin external preparation, a cosmetic and a food and drink composition for antiinflammation, comprising this anti-inflammatory agent.

A still further object of the present invention is to provide a novel skin barrier function-improving agent, derived from locust bean, which is capable of enhancing the expression of aquaporin 3 gene and/or filaggrin 2 gene. Further, an even further object is to provide a skin external preparation, a cosmetic and a food and drink composition for improving skin barrier function, comprising this skin barrier function-improving agent.

### Solution to Problem

The present inventors have separated a novel isoflavone compound from an extract of pods of locust bean, and have found that this novel compound has anti-inflammatory action as well as the effect of promoting the expression of aquaporin 3 gene and filaggrin 2 gene. Based on these findings, the present inventors have completed the present invention.

In order to solve the above problems, the present invention is a compound represented by the following formula (I) or a salt thereof, or a solvate thereof.

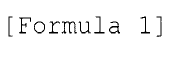

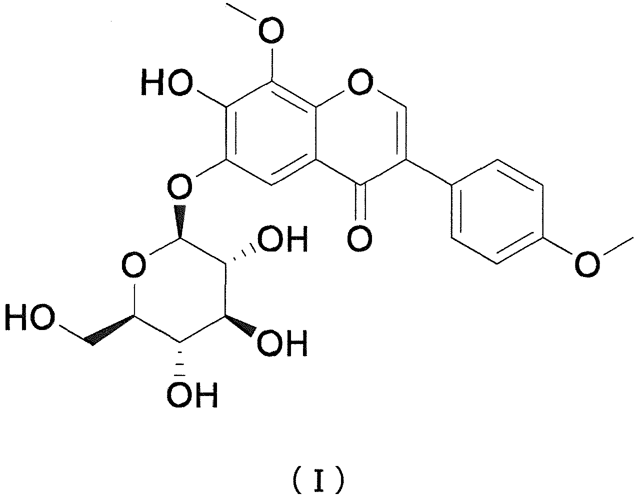

The compound represented by the formula (I) is a novel isoflavone compound obtained by separation and purification from the extract of pods of locust bean (*Ceratonia siliqua*)*.* It has an aquaporin-3 production-promoting action and a filaggrin 2 production-promoting action, as well as a skin barrier function-improving effect and an anti-inflammatory action.

The skin barrier function-improving agent of the present invention comprises a compound represented by the above formula (I) or a salt thereof, or a solvate thereof. Administration of the compound represented by the formula (I) promotes the expression of aquaporin 3 gene and filaggrin 2 gene and thereby promotes the production of aquaporin 3 and filaggrin 2 that are responsible for keeping the skin barrier function in an appropriate condition, so that the compound can exert a skin barrier function-improving effect.

The aquaporin 3 production-promoting agent of the present invention comprises a compound represented by the above formula (I) or a salt thereof, or a solvate thereof. Administration of the compound represented by the formula (I) promotes the expression of aquaporin 3 gene and thereby promotes the production of aquaporin 3.

The filaggrin 2 production-promoting agent of the present invention comprises a compound represented by the above formula (I) or a salt thereof, or a solvate thereof. Administration of the compound represented by the formula (I) promotes the expression of filaggrin 2 gene and thereby promotes the production of filaggrin 2.

The anti-inflammatory agent of the present invention comprises a compound represented by the above formula (I) or a salt thereof, or a solvate thereof. Administration of the compound represented by the formula (I) inhibits the production of IL-6 that is an inflammatory factor and thereby reduces the inflammatory response.

In the skin barrier function-improving agent, aquaporin 3 production-promoting agent, filaggrin 2 production-promoting agent and anti-inflammatory agent of the present invention, the concentration of the compound represented by the above formula (I) or a salt thereof, or a solvate thereof is also preferably 0.001 mM to 1 mM. This enables the concentration of the active ingredient, having an excellent effect, to be selected.

The skin barrier function-improving agent, aquaporin 3 production-promoting agent, filaggrin 2 production-promoting agent and anti-inflammatory agent of the present invention are also preferably a skin external preparation or cosmetic. Thereby, it is possible to obtain a skin external preparation or cosmetic having a skin barrier function-improving effect, an aquaporin 3 production-promoting action, a filaggrin 2 production-promoting action and an anti-inflammatory action.

The food and drink composition for improving skin barrier function of the present invention comprises a compound represented by the above formula (I) or a salt thereof, or a solvate thereof. Thereby, it is possible to obtain a food and drink composition that exerts a skin barrier function-improving effect through enhancing the expression of aquaporin 3 gene and filaggrin 2 gene and promoting the production of aquaporin 3 and filaggrin 2 and as a result, exerts a skin barrier function-improving effect.

The food and drink composition for antiinflammation of the present invention comprises a compound represented by the above formula (I) or a salt thereof, or a solvate thereof. Thereby, it is possible to obtain a food and drink composition that exerts an anti-inflammatory action through inhibiting the production of IL-6 that is an inflammatory factor.

A method for producing a compound represented by the above formula (I) comprises: obtaining a hydroalcoholic extract of pods of locust bean (*Ceratonia siliqua*); subjecting the hydroalcoholic extract to liquid-liquid extraction with petroleum ether and ethyl acetate in this order to collect ethyl acetate fractions; and separating the compound represented by formula (I) from the collected ethyl acetate fractions. By the method, a novel isoflavone compound having an aquaporin 3 production-promoting action and filaggrin 2 production-promoting action as well as a skin barrier function-improving effect and an anti-inflammatory action can be obtained.

### Advantageous Effects of Invention

The present invention can provide a novel isoflavone compound as well as a skin barrier function improving agent, an aquaporin 3 production-promoting agent, a filaggrin 2 production-promoting agent, an anti-inflammatory agent, a skin external preparation, a cosmetic and a food and drink composition comprising the compound, having the following excellent effects.
(1) The isoflavone compound can enhance the expression of aquaporin 3 gene and thereby promote the production of aquaporin 3.
(2) The isoflavone compound can enhance the expression of filaggrin 2 gene and thereby promote the production of filaggrin 2.
(3) The isoflavone compound has an aquaporin 3 production-promoting action and filaggrin 2 production-promoting action, and a synergistic skin barrier function-improving effect.
(4) The isoflavone compound inhibits IL-6 production and thereby has an anti-inflammatory action.
(5) They are highly safe for the human body, because they have, as an active ingredient, a compound derived from pods of locust bean that have been edible since ancient times.

### Brief Description of Drawings

[Figure 1] Figure 1 is a diagram showing a high-resolution ESI mass spectrum of the compound of the present invention.
[Figure 2] Figure 2 is a diagram showing an ultraviolet absorption spectrum of the compound of the present invention.
[Figure 3] Figure 3 is a diagram showing an infrared absorption spectrum of the compound of the present invention.
[Figure 4] Figure 4 is a diagram showing a ¹H-NMR spectrum (CD₃OD, 600 MHz) of the compound of the present invention.
[Figure 5] Figure 5 is a diagram showing a ¹³C-NMR spectrum (CD₃OD, 150 MHz) of the compound of the present invention.
[Figure 6] Figure 6 is a diagram showing a HSQC spectrum of the compound of the present invention.
[Figure 7] Figure 7 is a diagram showing a HMBC spectrum of the compound of the present invention.
[Figure 8] Figure 8 is a diagram showing a NOESY spectrum of the compound of the present invention.
[Figure 9] Figure 9 is a summary of ¹H-NMR signals and ¹³C-NMR signals of the compound of the present invention.
[Figure 10] Figure 10 is a diagram showing the HMBC correlation and the NOESY correlation in the compound of the present invention.
[Figure 11] Figure 11 is a graph indicating the amount of IL-6 produced by LPS induction in Example 4.
[Figure 12] Figure 12 is a graph showing the expression level of mRNA of the aquaporin 3 gene (AQP3) in Example 5.
[Figure 13] Figure 13 is a graph showing the expression level of mRNA of the filaggrin 2 gene (FLG2) in Example 5.

### Description of Embodiments

Hereinafter, a novel isoflavone compound of the present invention; a skin barrier function improving agent, an aquaporin 3 production-promoting agent, a filaggrin 2 production-promoting agent, an anti-inflammatory agent, a skin external preparation, a cosmetic, a food and drink composition for improving the skin barrier function and a food and drink composition for antiinflammation comprising the compound; and a method for producing the compound will be described.

The novel isoflavone compound represented by the following formula (I) according to the present invention is an isoflavone glycoside having an isoflavone compound and glucose ester-bonded.

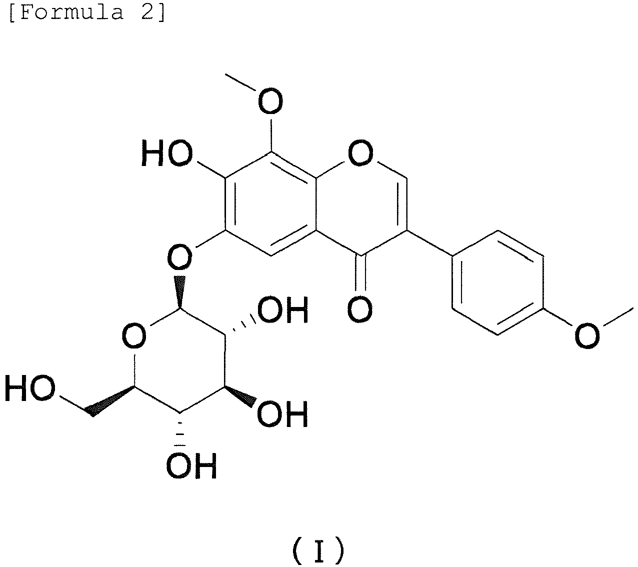

The novel isoflavone compound according to the present invention may be a salt thereof, and is preferably a pharmacologically acceptable salt. The pharmacologically acceptable salt of the novel isoflavone compound may be any salt formed together with an acid or base without limitation. The novel polyphenol compound or a salt thereof may be a solvate thereof. Examples thereof include, but is not particularly limited thereto, a hydrate and an organic solvate such as a solvate with ethanol.

The novel isoflavone compound according to the present invention has both an excellent aquaporin 3 production-promoting action and filaggrin 2 production-promoting action, and can be therefore used as a skin barrier function-improving agent that provides the effects of normalizing the skin barrier and keeping the barrier function by multiple mechanisms. The compound of the present invention also has IL-6 production inhibitory activity and can be used as an anti-inflammatory agent.

In the context of the present invention, the term "aquaporin 3 production-promoting" refers to promoting the expression of aquaporin 3 gene or promoting the expression of aquaporin 3 (protein), and it means that the expression of the aquaporin 3 gene or aquaporin 3 is enhanced compared to the control without the addition or administration of the novel isoflavone compound of the present invention. More specifically, the expression level of aquaporin 3 gene is preferably 1.5 times or more that of the control, more preferably 1.7 times or more, and particularly preferably 2 times or more. As used herein, the term "filaggrin 2 production-promoting" refers to promoting the expression of filaggrin 2 gene or promoting the expression of filaggrin 2 (protein), and it means that the expression of the filaggrin 2 gene or filaggrin 2 is enhanced compared to the control without the novel isoflavone compound of the present invention added or administered. More specifically, the expression level of filaggrin 2 gene is preferably 1.1 times or more that of the control, more preferably 1.2 times or more, and particularly preferably 1.3 times or more. The expression level of each of these genes can be measured by any known method such as real-time PCR (QPCR) or microarray, and the expression level of each of these proteins can be measured by any known method such as immunostaining and Western blotting.

In the context of the present invention, the term "anti-inflammatory action" means, for example, an action of reducing the amount of IL-6 produced by LPS induction, as compared to a control without the addition or administration of the novel isoflavone compound of the present invention.

The novel isoflavone compound of the present invention can be obtained by separation and purification from pods of locust bean. The locust bean used in the present invention has a scientific name of *Ceratonia siliqua*, and is a plant belonging to the family Fabaceae, the subfamily Caesalpinioideae, the genus Ceratonia. It is a plant mainly native to the Mediterranean region, but in the present invention, the production area and cultivation environment are not particularly limited and locust bean from any production area and cultivation environment can be used.

A method for separating the novel isoflavone compound of the present invention from locust bean will be described. First, a hydroalcoholic extract is obtained from pods of locust bean. In the context of the present invention, "hydroalcoholic extract of pods of locust bean" refers to an extract obtained by subjecting pods of locust bean to an extraction treatment by adding a hydrous alcohol as an extraction solvent. The pod of locust bean refers to a shell and fruits of "fruits with a shell of locust bean", and either one of the shell or the fruits can be used as an extraction material, but both the shell and the fruits are more preferably used. The extraction treatment is subjected to the as-harvested, i.e., fresh or dried pods of locust bean. However, the extraction treatment can be subjected to the pods of locust bean that have been subjected to various pretreatment in order to improve extraction efficiency or facilitate handling. Examples of the pretreatment include, but are not particularly limited to, drying treatment, crushing treatment and grinding treatment. The pods of locust bean that have been subjected to such a pretreatment may be also subjected to the extraction treatment to obtain an extract.

The alcohol constituting the hydrous alcohol used as an extraction solvent is not particularly limited as long as it can extract the isoflavone compound of the present invention. Examples of such an alcohol include ethanol, methanol, propanol, isopropanol, butanol and isobutanol. Among these, hydrous ethanol is preferably selected as an extraction solvent from the viewpoint of safety to the human body, extraction efficiency, and the like. The concentration of the hydrous alcohol is preferably 50 to 99%, more preferably 60 to 97%, and particularly preferably 70 to 95%. The extraction solvent can further comprise other ingredients unless they interfere with the extraction of the compound of the present invention.

The method of extracting with a hydrous alcohol is a method comprising extracting pods of locust bean by adding the hydrous alcohol as an extraction solvent to the pods and immersing the pods in the hydrous alcohol. For example, when the pods of locust bean are dry crushed material with a water content of less than 10%, the extraction solvent is preferably used in the amount of 5 to 10 parts by weight per 1 part by weight of the plant body. The extraction method may be any method such as extraction at room temperature, extraction by heating, extraction by pressurizing and heating, or subcritical extraction, but extraction by heating under reflux is preferable from the viewpoint of extraction efficiency. In order to increase the extraction efficiency, the extraction operation is preferably performed multiple times, and more preferably performed multiple times with different alcohol concentrations in the extraction solvent. Examples of the extraction method include, but are not particularly limited to, an extraction method comprising performing extraction under reflux with 95% ethanol one to five times followed by extraction under reflux with 70% ethanol one to five times. The extraction time is variously set, depending on the extraction method, the type of extraction material, the type of extraction solvent or the extraction temperature or the like. However, for example, when the extraction under reflux is performed with 70 to 95% ethanol, the extraction time for one extraction is preferably set to about 1 to 3 hours, and particularly preferably about 1.5 hours. After the extraction treatment described above, the residue is removed by decantation, centrifugation or filtration, or the like to obtain a hydroalcoholic extract of pods of locust bean. The obtained extract also includes a concentrate or a solid obtained by subjecting the extract to a treatment such as distillation under reduced pressure.

The hydroalcoholic extract of pods of locust bean obtained as described above may contain saccharides and the like that are contained in large amounts in the pods of locust bean. Therefore, for the purpose of removing these unnecessary components, the hydroalcoholic extract may be subjected to separation operation with an ion exchange resin. Specifically, 1 to 10 parts by weight of water is added to 1 part by weight of a hydroalcoholic extract of pods of locust bean and disperse the extract in water. The dispersion is then passed through a column packed with an ion exchange resin such as a macroporous adsorption resin to adsorb the isoflavone compound of the present invention and flow out and remove unnecessary components such as saccharides. Thereafter, the adsorbed isoflavone compound of the present invention is eluted with 95% ethanol or the like to collect fractions containing the isoflavone compound of the present invention.

Next, a further separation operation to which the hydroalcoholic extract of pods of locust bean or the collected fractions separated by the ion exchange resin described above are subjected will be described. The hydroalcoholic extract or the collected fractions are dispersed in an aqueous solvent and are subjected to solvent extraction with petroleum ether and ethyl acetate in this order. The aqueous solvent is not particularly limited as long as it can disperse the isoflavone compound of the present invention, but a 50% hydrous methanol is suitably used. After performing liquid-liquid extraction with petroleum ether/an aqueous solvent multiple times, liquid-liquid extraction with ethyl acetate/an aqueous solvent is performed multiple times. The ethyl acetate fractions collected by this solvent extraction operation contain the novel isoflavone compound of the present invention. The number of liquid-liquid extractions in each solvent system is preferably about 2 to 10, particularly preferably about 5.

The methyl acetate fractions obtained as described above can be purified by a conventional method to isolate the novel isoflavone compound of the present invention. Examples of the purification method includes normal phase chromatography, reverse phase chromatography, thin layer chromatography, gel filtration chromatography and high-performance liquid chromatography. Purification may be performed by using one of these chromatographies or two or more in combination. Carriers, elution solvents and the like used in each chromatography can be appropriately selected depending on the chromatography.

The isoflavone compound of the present invention which has been separated and purified from pods of locus bean as described above may not be isolated as a completely pure substance, and may contain some other components derived from a locust bean raw material.

The novel isoflavone compound of the present invention may be a synthetic product synthetically obtained by any known method.

The novel isoflavone compound of the present invention has both an excellent aquaporin 3 production-promoting action and filaggrin 2 production-promoting action, and can be therefore used as a skin barrier function-improving agent by multiple mechanisms. The novel isoflavone compound of the present invention can be also used as an aquaporin 3 production-promoting agent or a filaggrin 2 production-promoting agent. The novel isoflavone compound of the present invention has IL-6 production inhibitory activity and can be therefore also used as an anti-inflammatory agent. The novel isoflavone compound of the present invention can be also used as an IL-6-production-inhibiting agent.

The skin barrier function-improving agent, aquaporin 3 production-promoting agent and filaggrin 2 production-promoting agent comprising the novel isoflavone compound of the present invention can be used as a skin external preparation for increasing the amounts of aquaporin 3 and filaggrin 2 contained in the skin cells, improving the skin barrier function, improving aging of the skin and keeping the skin in a stable condition. The skin barrier function-improving agent, aquaporin 3 production-promoting agent and filaggrin 2 production-promoting agent of the present invention can be also used as a cosmetic for increasing the amounts of aquaporin 3 and filaggrin 2 contained in the skin cells, keeping the skin moisture and keeping the skin in a healthy condition. The anti-inflammatory agent comprising a novel isoflavone compound of the present invention can be also used as a skin external preparation for inhibiting the production of an inflammatory factor, preventing or improving inflammation in the skin and keeping the skin in a stable condition. The anti-inflammatory agent of the present invention can be also used as a cosmetic for inhibiting the production of an inflammatory factor and keeping the skin in a healthy condition.

The dosage of skin barrier function-improving agent, aquaporin 3 production-promoting agent, filaggrin 2 production-promoting agent and anti-inflammatory agent of the present invention varies depending on the target effect, preventive or therapeutic effect, administration method or age, and cannot be determined in general. However, when used as an external preparation, the usual daily parenteral dosage is preferably 0.2 **µ**g to 30 mg, more preferably 1 **µ**g to 3 mg, and even more preferably 5 **µ**g to 500 **µ**g of the isoflavone compound of the present invention. When used as an internal preparation, the usual daily oral dosage is preferably 1 **µ**g to 1000 mg, and more preferably 5 **µ**g to 500 mg of the isoflavone compound of the present invention.

The dosage form of the skin barrier function-improving agent, aquaporin 3 production-promoting agent, filaggrin 2 production-promoting agent and anti-inflammatory agent as well as the skin external preparation and cosmetic of the present invention is not particularly limited, and examples thereof include liquids such as low-viscosity liquids or lotions; emulsions, gels, pastes, creams, foams, packs, ointments, powders, aerosols and patches as well as tablets, granules, capsules and liquids for internal application. The skin barrier function-improving agent, aquaporin 3 production-promoting agent, filaggrin 2 production-promoting agent and anti-inflammatory agent according to the present invention can be applied for any of a cosmetic, a quasi-drug and a pharmaceutical. Specific examples of the cosmetic, quasi-drug and pharmaceutical include, but are not particularly limited to, cosmetic lotions, cosmetic creams, cosmetic emulsions, serums, cosmetic packs, cosmetic cleansers, soaps, hair care agents, bath agents, makeup cosmetics, and anti-acne skin care cosmetics.

The blending concentration of the novel isoflavone compound of the present invention comprised in the skin barrier function-improving agent, aquaporin 3 production-promoting agent, filaggrin 2 production-promoting agent and anti-inflammatory agent as well as the skin external preparation and cosmetic of the present invention is preferably 0.001 mM to 1 mM, more preferably 5 **µ**M to 100 **µ**M, and even more preferably 10 **µ**M to 30 **µ**M. When the amount of the novel isoflavone compound of the present invention is within this range, the compound can be stably blended, it can be highly safe for the skin and it can exert a high skin barrier function-improving effect, aquaporin 3 production-promoting effect, filaggrin 2 production-promoting effect and anti-inflammatory effect.

The skin barrier function-improving agent, aquaporin 3 production-promoting agent, filaggrin 2 production-promoting agent and anti-inflammatory agent of the present invention can be prepared in various forms by any method conventionally used. In this case, it can be formulated with additives that are usually accepted as pharmaceutical additives, such as carriers and excipients for formulations. In addition, in order to improve the bioavailability and stability of the present compound, a drug delivery system including a formulation technique such as microencapsulation, pulverization or inclusion with cyclodextrin or the like can be also used.

The skin barrier function-improving agent, aquaporin 3 production-promoting agent, filaggrin 2 production-promoting agent and anti-inflammatory agent as well as the skin external preparation and the cosmetic of the present invention can comprise appropriately an ingredients commonly used in an external skin preparations and cosmetics, such as water, fats and oils, waxes, hydrocarbons, fatty acids, higher alcohols, esters, plant extracts, vitamins, water-soluble polymers, surfactants, metallic soaps, alcohols, polyhydric alcohols, pH adjustors, preservatives, fragrances, powders, thickeners, pigments and chelating agents. In addition, one or two or more of various functional ingredients usually used, for example, selected from moisturizing agents, whitening agents, anti-oxidant agents, cell activators, ultraviolet protection agents, blood circulation promoters and other anti-inflammatory agent and the like can be used in combination with the compound of the present invention, unless they impair the actions and effects of the present invention.

The food and drink composition for improving skin barrier function and the food and drink composition for antiinflammation of the present invention also comprises, as an active ingredient, the novel isoflavone compound of the present invention. The food and drink composition according to the present invention can be prepared in any form, for example, in the form of supplements such as tablets, capsules, granules and syrups; and drinks such as soft drinks, fruit juice drinks, and alcoholic drinks; sweets such as candies, gums, cookies, biscuits and chocolates; breads, porridges, cereals, noodles, jellies, soups, dairy products and seasonings. When used as food or drink products in this way, the food and drink composition can be combined in various manner with various other active ingredients, or nutrients such as vitamins, minerals and amino acids unless they affect the efficacy of the active ingredient of the present invention. The food and drink product of the present invention include supplements, health foods, functional foods, foods for specified health uses, and the like. The daily intake of the food and drink product of the present invention is preferably 1 **µ**g to 1000 mg and more preferably 5 **µ**g to 200 mg of the isoflavone compound of the present invention.

Hereinafter, the present invention will be described in detail with reference to examples thereof, but the present invention will not be limited by these examples.

### Examples

### [Example 1]

### 1. Preparation of hydroalcoholic extract of pods of locust bean.

Seeds were removed from fruits with shells of locust bean (*Ceratonia siliqua*) that had been subjected to drying treatment after harvesting. Pods of this locust bean was ground with a grinder to obtain a ground product having a particle size of 2 mm or less. The operation of adding, to 20 kg of the ground product, 140 kg (7 times the amount of the ground product) of a 95% hydrous ethanol and extracting under refluxing for 1.5 hours was performed twice. Thereafter, the residue was further extracted under reflux with 140 kg (7 times the amount of the ground product) of a 70% hydrous ethanol for 1.5 hours. The extracts obtained by extraction under reflux were combined, and the solvent was then distilled off under reduced pressure to obtain 12.4 kg of a hydrous ethanol extract of pods of locust bean.

### [Example 2]

### 2. Separation and purification of hydroalcoholic extract of pods of locust bean

The hydroalcoholic extract of pods of locust bean obtained in Example 1 was dispersed in 1 to 10 times the amount of water and adsorbed on an ion exchange resin (a macroporous adsorption resin D101, available from Cangzhou Bon Adsorber Technology Co., Ltd.). Impurities such as saccharides were removed by eluting them with 3 times the column volume of distilled water, elution was then performed with 3 times the column volume of a 95% hydrous ethanol, and the solvent was distilled off under reduced pressure to obtain 462.7 g of ethanol elution fractions (non-carbohydrate-based low molecular compound fractions). Next, the obtained ethanol elution fractions were dispersed in 1.0 L of 50% hydrous methanol, and the dispersion was subjected to liquid-liquid extraction with petroleum ether and ethyl acetate in this order each five times. Each solvent was distilled off under reduced pressure to obtain 28.4 g of petroleum ether fractions, 139.4 g of ethyl acetate fractions and 290.2 g of aqueous fractions, respectively.

Then, a 135.0 g portion of the ethyl acetate fractions was subjected to normal phase silica gel column chromatography (a column packing material: 200 to 300 mesh; a product available from Qingdao Haiyang Chemical Co., Ltd.), and elution and fractionation were performed with two developing solvents, that is, petroleum ether (P)/ethyl acetate (E) and dichloromethane (C)/methanol (M). As a result, 108 elution fractions were obtained. The obtained 108 elution fractions were subjected to identification with thin layer chromatography (a silica gel GF₂₅₄ plate; a product available from Qingdao Haiyang Chemical Co., Ltd.), and similar fractions were combined to obtain ten elution fractions A to J.

Then, the elution fraction I (20.0 g) was subjected to reverse phase ODS column chromatography (a column packing material: 40 to 63 **µ**m; a product available from Merck & Co.), and fractionation was performed by gradient elution with methanol: water = 15:85 → 100:0. The obtained elution fractions were subjected to identification with thin layer chromatography (a silica gel GF₂₅₄ plate; a product available from Qingdao Haiyang Chemical Co., Ltd.), and similar fractions were combined to obtain five elution fractions I1 to I5.

Next, the elution fraction I5 (2.7 g) was subjected to gel filtration chromatography (a column packing material: Sephadex LH-20), and elution was performed with dichloromethane: methanol = 1:1 to obtain five elution fractions I5a to I5e.

Then, the elution fraction I5c (0.3 g) was subjected to semi-preparative HPLC (Column I: YMC-Pack ODS-A, 250 × 20 mm, 5 µm), and separation was performed with methanol:water = 55:45 at a detection wavelength of 267 nm to obtain a fraction I5cl. This fraction I5c1 was subjected to semi-preparative HPLC (Column II: YMC-Pack ODS-A, 250 × 10 mm, 5 µm), and separation was performed with methanol:water = 49:51 at a detection wavelength of 267 nm to obtain 3.3 mg of a compound of the present invention (hereinafter referred to as "Ceratonia siliqua C") (retention time tR = 56.42 minutes).

### [Example 3]

### 3. Structural analysis of compound (Ceratonia siliqua C)

The compound obtained in Example 2, "Ceratonia siliqua C" was subjected to structural analysis. For structural analysis, high-resolution mass spectrometry (HR-ESI-MS; positive ion mode), ultraviolet absorption spectrometry, infrared absorption spectrometry and ¹H-NMR, ¹³C-NMR, HMBC, HSQC and NOESY analyses were performed. The following devices were used for these analyses.
- High-resolution mass spectrometry: an electrospray ionization quadrupole time-of-flight mass spectrometry device (a product available from Bruker Daltonics)
- Ultraviolet absorption spectrometry: an ultraviolet-visible spectrophotometer (UV-2401PC, a product available from Shimadzu Corporation)
- Infrared absorption spectrometry: FT-IR (NEXUS470, a product available from Thermo nicolet)
- NMR analysis: a 600 MHz nuclear magnetic resonance device (AVANCE III 600, a product available from Bruker)

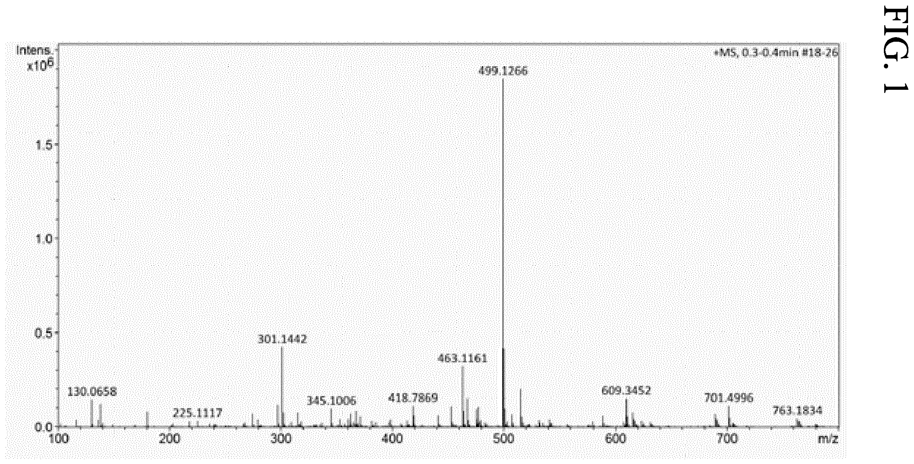

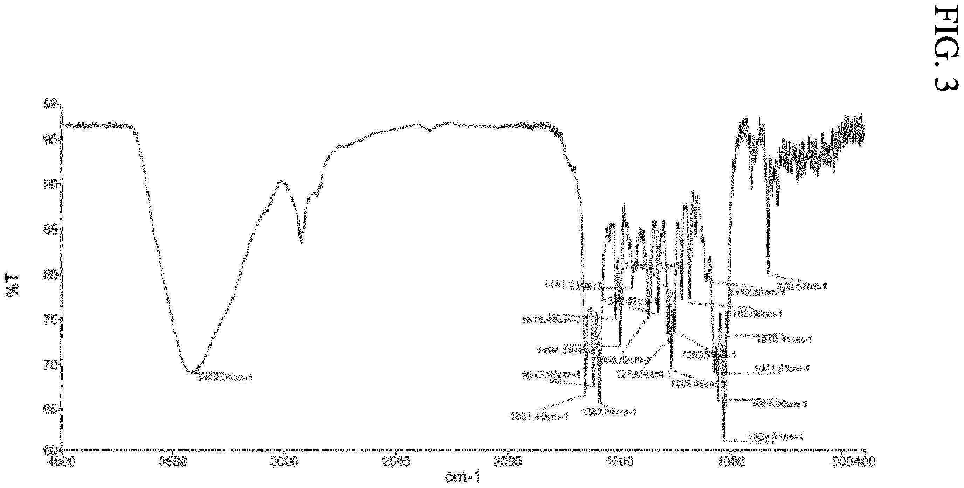

The physical properties of Ceratonia siliqua C are as follows. The spectrum of high-resolution mass spectrometry (HR-ESI-MS) is shown in Figure 1, the results of ultraviolet absorption spectrometry are shown in Figure 2, and the results of infrared absorption spectrometry are shown in Figure 3.
- Aspect: yellow powder
- HR-ESI-MS (positive) m/z: 499.1266 [M + Na]⁺
- Ultraviolet absorption spectrum: λmax (MeOH) nm (log ε) : 204.6 (4.07), 264.2 (4.02)
- Infrared absorption spectrum (KBr, cm⁻¹): vmax: 3422.30, 1651.40, 1613.95, 1587.91, 1516.46, 1494.55, 1441.21, 1366.52, 1323.41, 1279.56, 1265.05, 1253.99, 1219.53, 1182.66, 1112.36, 1071.83, 1055.90, 1029.91, 1012.41, 830.57

The results of high-resolution mass spectrometry (Figure 1) showed that the quasi-molecular ion peak was m/z 499.1266 [M + Na]⁺. Therefore, the compositional formula was estimated to be C₂₃H₂₄O₁₁, and the degree of unsaturation was estimated to be 12. The ultraviolet absorption spectrum (Figure 2) showed maximum absorption at 204 nm and 264 nm, suggesting the possibility that Ceratonia siliqua C would be a flavonoid compound. The infrared absorption spectrum (Figure 3) showed that the molecular structure included functional groups such as a hydroxyl group (3422.30 cm⁻¹), a benzene ring (1613.95 cm⁻¹, 1494.55 cm⁻¹) and a ketocarbonyl group (1651.40 cm⁻¹), and the like.

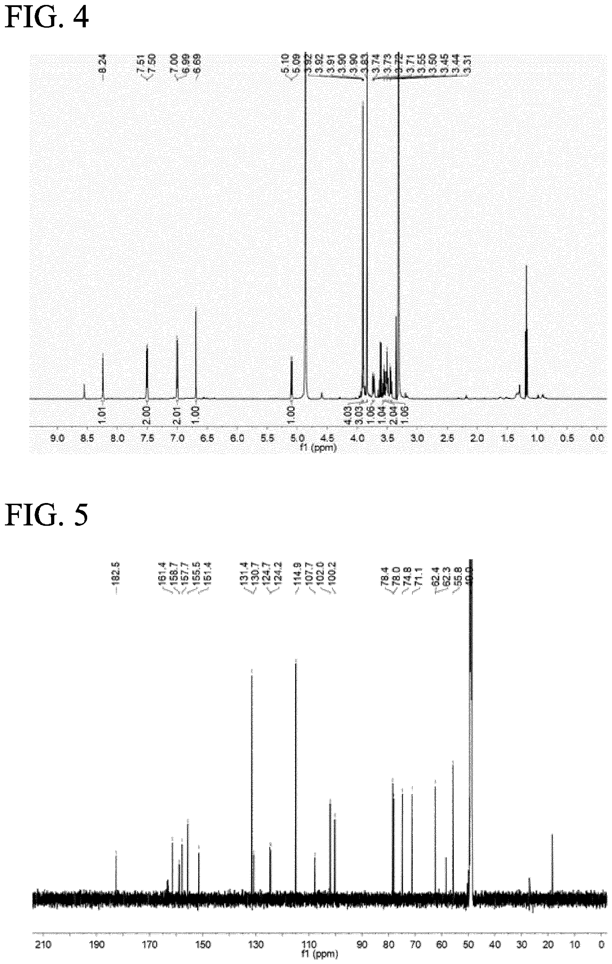

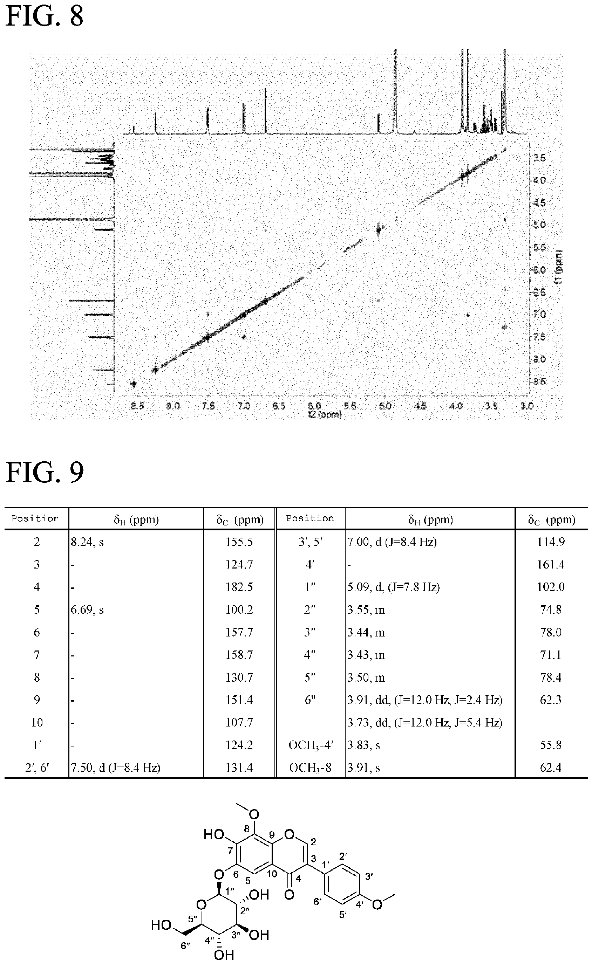

From the spectrum obtained by ¹H-NMR analysis (a solvent: CD₃OD, observation frequency: 600 MHz) (Figure 4), the following was confirmed (Figure 9): two aromatic singlet signals [**δ**_{H}: 8.24 (1H, s, H-2), 6.69 (1H, s, H-5]; a set of AABB system proton signals [**δ**_{H}: 7.50 (2H, d, J = 8.4 Hz, H-2', H-6'), 7.00 (2H, d, J = 8.4 Hz, H -3', H-5')]; two methoxy group signals [**δ**_{H}: 3.91 (3H, s, OCH₃-8), 3.83 (3H, s, OCH₃-4')]; a set of glucose residue proton signals [**δ**_{H}: 5.09 (1H, d, J = 7.8 Hz, H-1"), 3.91 (1H, dd, J = 12.0 Hz, J = 2. 4 Hz, H-6**α**"), 3.73 (1H, dd, J = 12.0 Hz, J = 5.4 Hz, H-6**β**"), 3.55 (1H, m, H-2"), 3. 50 (2H, m, H-3", H-5"), 3.43 (1H, m, H-4")]. From the spectrum obtained by ¹³C-NMR analysis (a solvent: CD₃OD, observation frequency: 150 MHz) (Figure 5), 23 carbon signals were observed (Figure 9). They included the following: one ketocarbonyl group carbon signal (**δ**c: 182.5); 14 aromatic or olefinic carbon signals (**δ**c: 161.4, 158.7, 157.7, 155.5, 151.4, 131.4 × 2, 130.7, 124.7, 124.2, 114.9 × 2, 107.7, 100.2): two methoxy group signals (**δ**c: 62.4, 55.8); and a set of glucose residue carbon signals (**δ**c: 102.0, 78.4, 78.0, 74.8, 71.1, 62.3). From these analysis results, this compound was presumed to be a flavonoid glycoside.

In addition, an aromatic proton signal [**δ**_{H}: 8.24 (1H, s, H-2)] characteristic of isoflavone was observed in the low magnetic field region of the ¹H-NMR spectrum. Therefore, this compound was presumed to be an isoflavone compound.

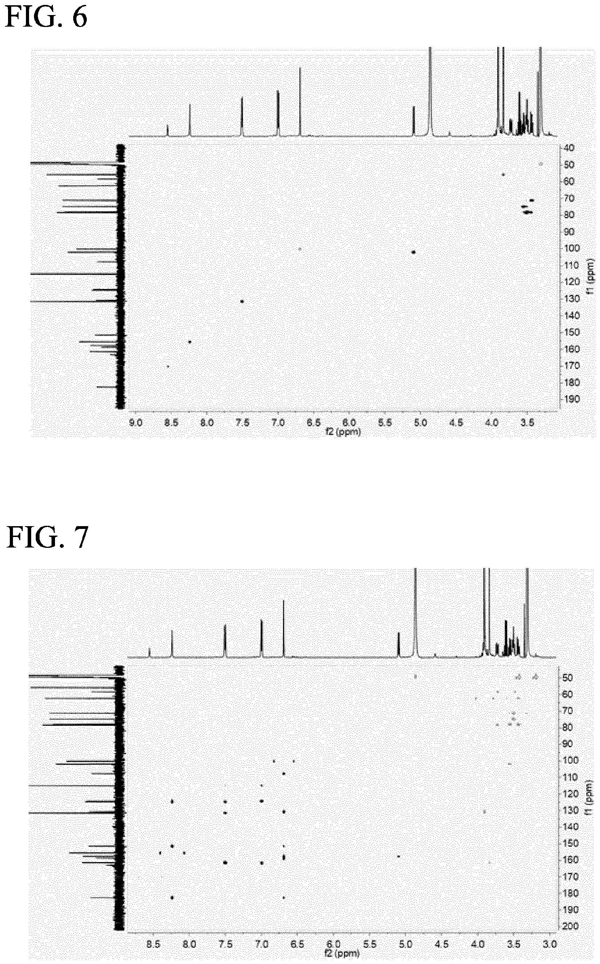

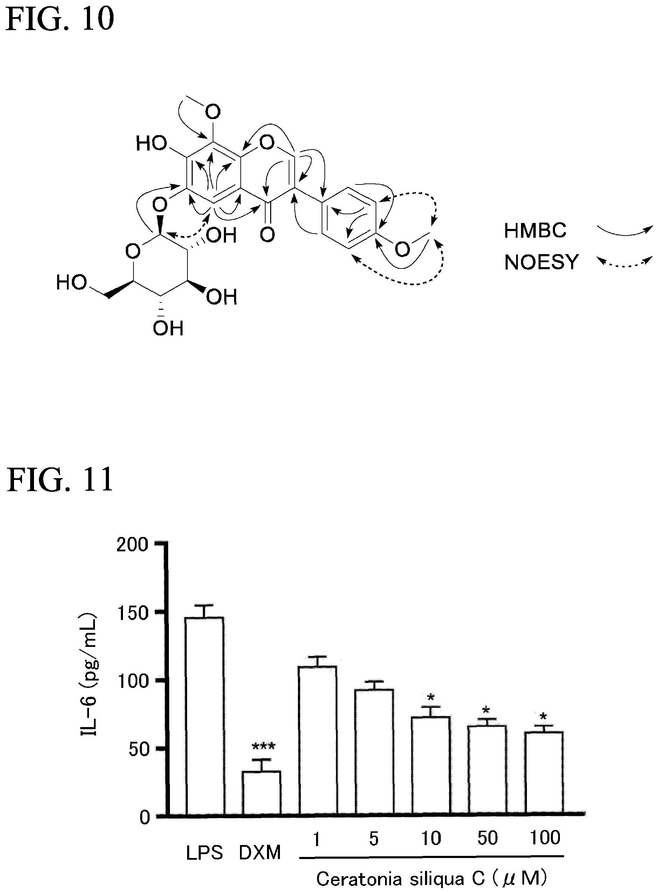

Further, from the spectrum obtained by HMBC analysis (Figure 7), long-range correlation signals were confirmed between H-2 and C-4/C-9/C-1', between H-2'/H-6' and C-3. This result showed that this compound was an isoflavone compound. The long-range correlation signals between H-2'/H-6' and C-4', OCH₃-4' indicate that OCH₃-4' is attached at the C-4' position. This was also confirmed by the correlation signals between H-3'/H-5' and OCH₃-4' observed in the NOESY spectrum (Fig. 8). In addition, from the HMBC spectrum, long-range correlation signals between H-5 and C-4/C-6/C-7/C-8/C-9 were observed, clearly indicating no substitution with a substituent at H-5 position. The long-range correlation signals between H-1" and C-6 indicated that the glucose residue was attached at the C-6 position. This was also confirmed by the correlation signals between H-1" and H-5 in the NOESY spectrum. Further, from the HMBC spectrum, long-range correlation signals were observed between OCH₃-8 and the carbon with a chemical shift value δ_{C}: 130.7, but long-range correlation signals were observed also between H-5 and C-7/C-8. Therefore, the attachment point of OCH₃-8 could not be confirmed in the HMBC spectrum. Accordingly, the structure of this compound and NMR data were examined with reference to a literature on flavonoid compounds (Yang G Y, Miao M M, Wang Y, et al., "Flavonoids from the leaves of Sun Cured Tobacco and their anti-tobacco mosaic virus activity", Cheminform., 2015, Vol.46, No. 6, p.1198-1203). The examination showed that the chemical shift value δ_{C} was about 130.0 when the C-8 position of the flavonoid compound was substituted with a methoxy group, and the chemical shift value δ_{C} was greater than 150.0 when the C-7 position is substituted with a methoxy group. Therefore, OCH₃-8 was identified to be attached at the C-8 position rather than the C-7 position. The coupling constant of protons at the glucose terminal was 7.8 Hz, indicating that the relative configuration of the glucose termini was of **β**-type. From these analysis results, this compound, Ceratonia siliqua C was determined to have a structure represented by the following formula (I). Figure 9 shows an assignment table summarizing **δ**_{H} of ¹H-NMR signals and δ_{C} of ¹³C-NMR signals based on the analyses of HSQC spectrum and HMBC spectrum. Figure 10 shows the analysis results of NOESY and HMBC for the Ceratonia siliqua C.

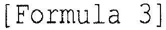

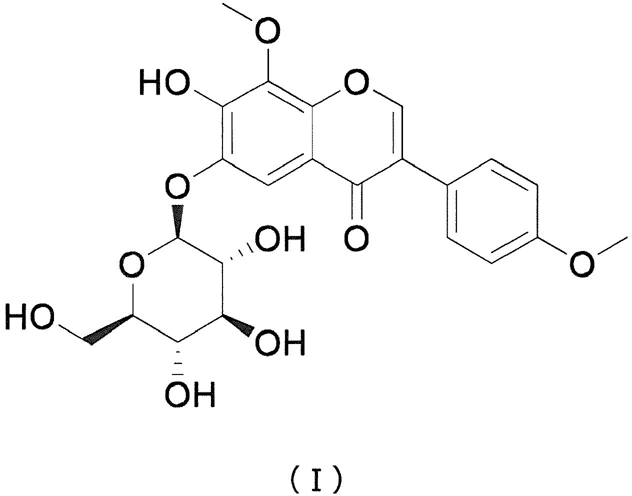

### [Example 4]

### 4. Anti-inflammatory action of Ceratonia siliqua C

The Ceratonia siliqua C obtained in Example 2 was used to examine the effect of the Ceratonia siliqua C on the production of IL-6 that is an inflammatory cytokine.

In this test, an RAW264.7 cell, a mouse macrophage-derived cell line was used. RAW264.7 cells were cultured in a DMEM medium (containing 10% FBS, 100 U/mL penicillin and 100 **µ**M/mL streptomycin) in the presence of 5% CO₂ at 37°C and saturated humidity.

Each well of a 96-well plate was seeded with 8 × 10³ RAW264.7 cells, and the cells were cultured in a CO₂ incubator for 24 hours. This was divided into a blank control group, a positive control group and a test group. A 100 **µ**L portion of a DMEM medium was added to each well of the blank control group; a 100 **µ**L portion of a solution of dexamethasone (DXM) dissolved in the DMEM medium was added to each well of the positive control group (a final concentration of dexamethasone: 20 **µ**M); and a 100 **µ**L portion of each of solutions obtained by appropriately diluting the Ceratonia siliqua C with the DMEM medium was added to each well of the test group to provide a different concentration from each other (final concentration: 1 **µ**M, 5 **µ**M, 10 **µ**M, 50 **µ**M and 100 **µ**M). 3 hours later, LPS (1 **µ**g/mL) was added to each well to induce inflammation. 24 hours later, the supernatant was collected and the IL-6 content in the supernatant was measured with an IL-6 quantitative ELISA kit. The test was performed in triplicate (3 wells) for the blank control group, for the positive control group and for each concentration of the sample solution in the test group (n = 3).

The results are shown in Figure 11. LPS represents a blank control and DXM represents a positive control (dexamethasone: 20 **µ**M). The compound of the present invention, the Ceratonia siliqua C was shown to inhibit the production of IL-6 in a concentration-dependent manner, indicating that the novel isoflavone compounds of the present invention had an anti-inflammatory action.

### [Example 5]

### 5. Effect of promoting the expression of skin barrier function-related genes of Ceratonia siliqua C

The Ceratonia siliqua C obtained in Example 2 was used to examine the effect of the Ceratonia siliqua C on the expression of aquaporin 3 (AQP3) gene and filaggrin 2 (FLG2) gene in a HaCaT cell, a human epidermis-derived keratinocyte cell line.

First, the HaCaT cells were cultured in a DMEM medium (containing 10% FBS, 100 U/mL penicillin and 100 **µ**M/mL streptomycin) in the presence of 5% CO₂ at 37°C and saturated humidity. Next, the HaCaT cells adjusted to a cell concentration of 5 × 10⁵ cells/mL were seeded in each well of a 6-well cell culture plate and cultured in a CO₂ incubator (5% CO₂, 37°C) for 24 hours. Thereafter, the Ceratonia siliqua C isolated in Example 2 was added to each well to provide a final concentration different from each other. Specifically, the final concentrations of Ceratonia siliqua C in the HaCaT cell culture medium were 1 **µ**M, 5 **µ**M, 10 **µ**M, 50 **µ**M and 100 **µ**M, respectively. A well without the Ceratonia siliqua C added was used as a control (0 **µ**M). After addition, they were subjected to culture for 24 hours.

After completion of the culture, the HaCaT cells were collected from each well, and total RNA was extracted with an RNA extraction reagent (RNAzol(R), a product available from Molecular Research Center, Inc.). The concentration of the total RNA obtained by extraction was confirmed with an ultratrace spectrophotometer (NanoDrop; a product available from Thermo Fisher Scientific). To this total RNA were added an oligo dT primer, a dNTP Mix, a 5 × 1st strand buffer, an RNase inhibitor, DTT and an M-MLV reverse transcriptase to synthesize cDNA from the total RNA. This cDNA was subjected to real-time PCR (QPCR) to measure the mRNA expression level of each of the aquaporin 3 gene and filaggrin 2 gene.

QPCR was performed with a commercially available QPCR reagent kit and QPCR measurement device (LightCycler(R) 480; a product available from Roche Diagnostics K. K.), and the detection was performed with SYBR(R)Green that is a double-stranded DNA-binding fluorescent dye. The primers of SEQ ID NOs: 1 and 2 shown in Table 1 below were used as a QPCR primer for aquaporin 3. The primers of SEQ ID NOs: 3 and 4 shown in Table 2 below were used as a QPCR primer for filaggrin 2.

**[Table 1]**

| | | |
|---|---|---|
| Forward primer | **5' - AGACAGCCCCTTCAGGATTT -3'** | SEQ ID NO: 1 |
| Reverse primer | **5' - TCCCTTGCCCTGAATATCTG -3'** | SEQ ID NO: 2 |

**[Table 2]**

| | | |
|---|---|---|
| Forward primer | **5' - CTGTGGTCATTCATGGAGTGG -3'** | SEQ ID NO: 3 |
| Reverse primer | **5' - CCCTAGAAGGGCTAATGTGTGA -3'** | SEQ ID NO: 4 |

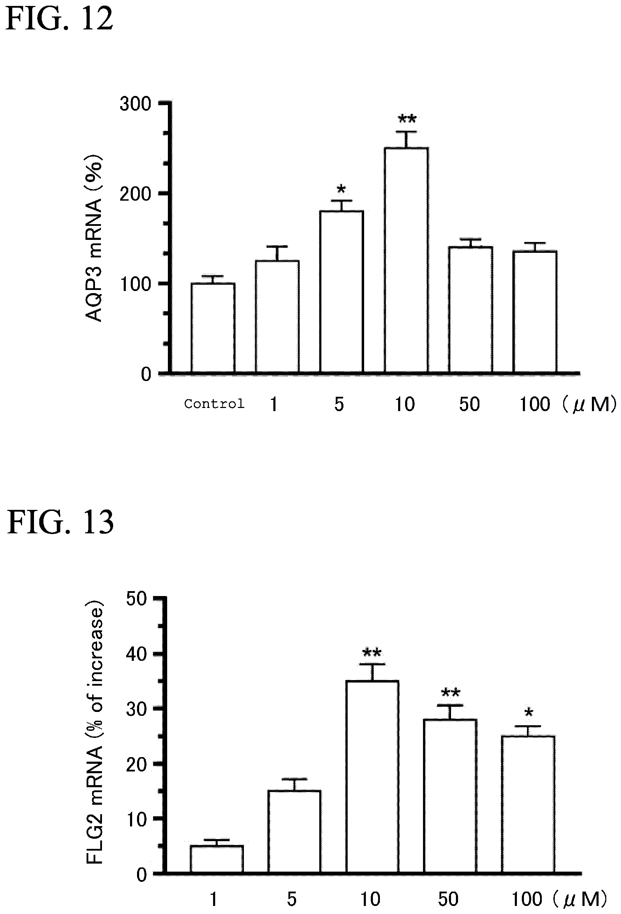

The results for aquaporin 3 are shown in Figure 12, and the results for filaggrin 2 are shown in Figure 13. The mRNA expression level of the aquaporin 3 is shown as a value relative to the expression level of a control of 100. The mRNA expression level of the filaggrin 2 is shown as an increase rate (%) relative to the expression level of a control. Figure 12 and Figure 13 clearly shows that the addition of the compound of the present invention, the Ceratonia siliqua C greatly increased the mRNA expression level of each of the aquaporin 3 and filaggrin 2 compared to the control without the Ceratonia siliqua C added. When the concentration of Ceratonia siliqua C was 10 **µ**M, the highest expression promoting effect was confirmed, and the mRNA expression level of aquaporin 3 was increased by 2.5 times and the mRNA expression level of filaggrin 2 was also increased by 1.35 times.

The present invention is not limited to the above-described embodiments or examples, and includes various modifications without deviating from the scope of the invention described in the claims.

### Industrial Applicability

The novel isoflavone compound of the present invention is used as a skin barrier function-improving agent, aquaporin 3 production-promoting agent, filaggrin 2 production-promoting agent, anti-inflammatory agent, skin external preparation or cosmetic, and is widely used in the medical and cosmetic fields.

### Sequence Listing Free Text

SEQ ID NO: 1: target gene: a forward primer for QPCR of aquaporin 3
SEQ ID NO: 2: target gene: a reverse primer for QPCR of aquaporin 3
SEQ ID NO: 3: target gene: a forward primer for QPCR of filaggrin 2
SEQ ID NO: 4: target gene: a reverse primer for QPCR of filaggrin 2

## Claims

1. A compound represented by the following formula (I) or a salt thereof, or a solvate thereof.

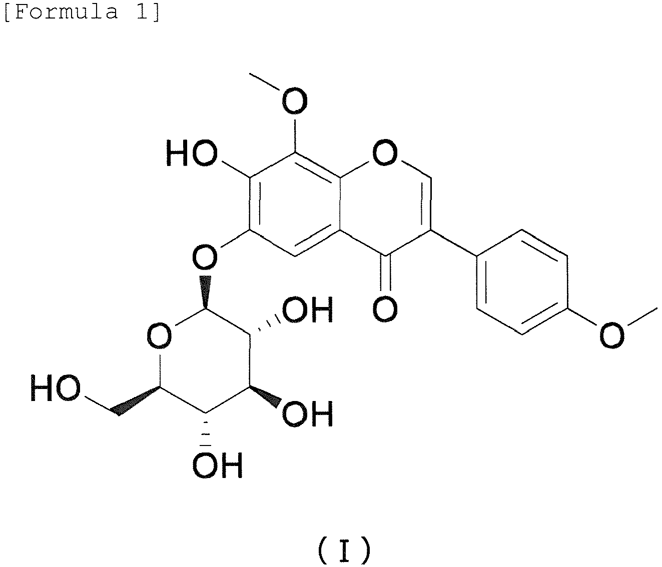

2. A skin barrier function-improving agent comprising a compound represented by the following formula (I) or a salt thereof, or a solvate thereof.

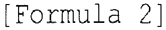

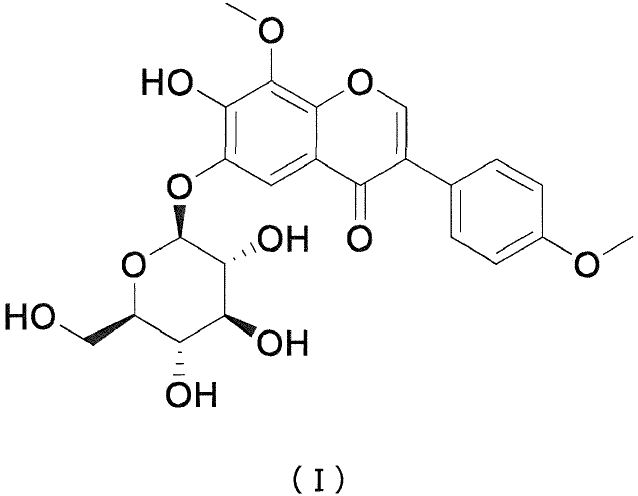

3. An aquaporin 3 production-promoting agent comprising a compound represented by the following formula (I) or a salt thereof, or a solvate thereof.

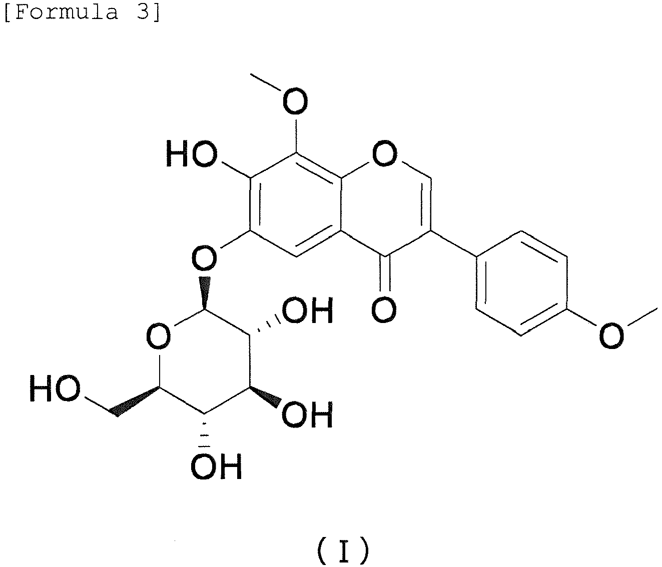

4. A filaggrin 2 production-promoting agent comprising a compound represented by the following formula (I) or a salt thereof, or a solvate thereof.

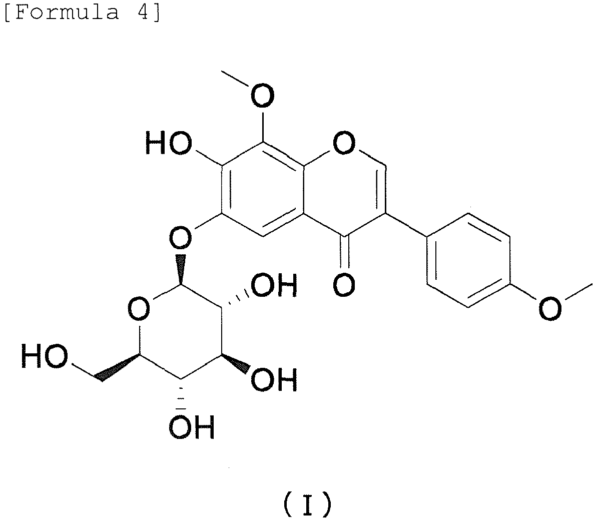

5. An anti-inflammatory agent comprising a compound represented by the following formula (I) or a salt thereof, or a solvate thereof.

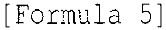

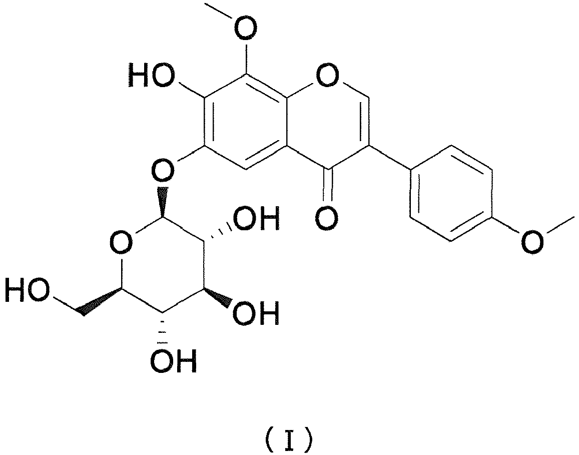

6. The agent according to any one of claims 2 to 5, wherein the concentration of the compound represented by the above formula (I) or a salt thereof, or a solvate thereof is 0.001 mM to 1 mM.

7. The agent according to any one of claims 2 to 5, which is a skin external preparation.

8. The agent according to any one of claims 2 to 5, which is a cosmetic.

9. A food and drink composition for improving skin barrier function comprising a compound represented by the following formula (I) or a salt thereof, or a solvate thereof.

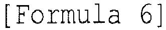

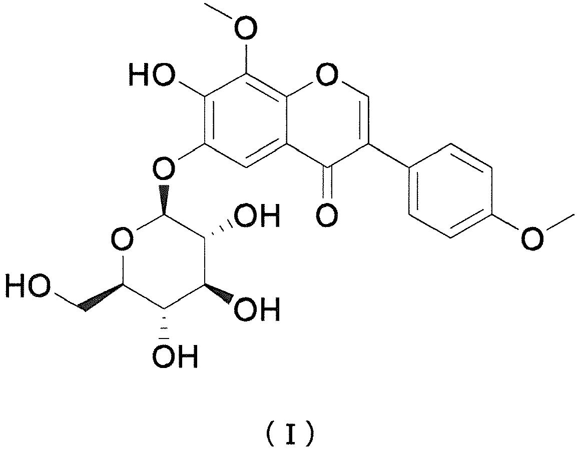

10. A food and drink composition for antiinflammation comprising a compound represented by the following formula (I) or a salt thereof, or a solvate thereof.

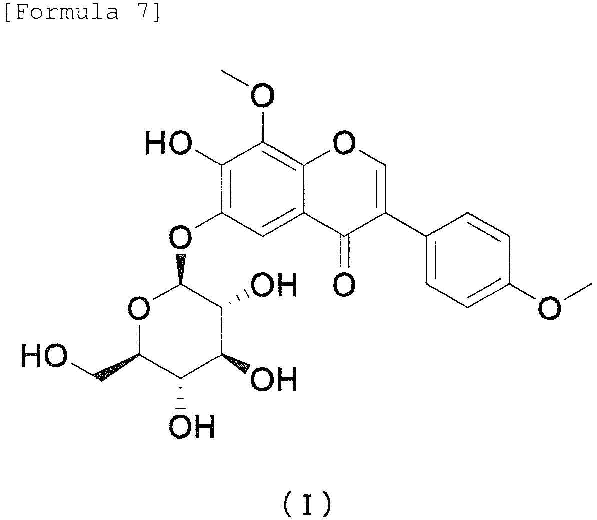

11. A method for producing a compound represented by the formula (I), comprising:
obtaining a hydroalcoholic extract of pods of locust bean (*Ceratonia siliqua*);
subjecting the hydroalcoholic extract to liquid-liquid extraction with petroleum ether and ethyl acetate in this order to collect ethyl acetate fractions; and
separating the compound from the ethyl acetate fractions.
